# FASCICULE DE BREVET EUROPEEN

(11) **EP 2 919 727 B1**
(45) Date de publication et mention de la délivrance du brevet: **23.08.2017**
(21) Numéro de dépôt: 13801606.8
(22) Date de dépôt: 08.11.2013
(51) Int. Cl.: A61F 9/02

(54) **MASQUE À ÉCRAN AMOVIBLE**
BRILLE MIT ABNEHMBARER BLENDE
GOGGLES WITH A DETACHABLE SHIELD

(30) Priorité: 13.11.2012 FR 1203036
(43) Date de publication de la demande: 23.09.2015
(73) Titulaire: Salomon S.A.S., 74370 Metz-Tessy (FR)
(72) Inventeur: FAVRE-FELIX, Hervé, 74370 Villaz (FR); BOCCHER, Eric, 74000 Annecy (FR); LAPERRIERE, Christophe, 74000 Annecy (FR)
(74) Mandataire: Lapierre, Stéphane
(86) Numéro de dépôt international: PCT/FR2013/000289
(87) Numéro de publication internationale: WO 2014/076377

(56) Documents cités:
- EP-A2- 1 702 596
- US-A1- 2008 155 736
- US-A1- 2011 225 709

## Description

L'invention concerne les masques de protection oculaire, en particulier les masques de protection pour la pratique de sports en plein air, tels que le ski ou surf alpin, le vélo tout terrain ou le motocross.

Des masques de protection sont habituellement utilisés durant la pratique de certains sports de plein air pour protéger les yeux de l'utilisateur du vent, de l'eau, de la boue ou du rayonnement solaire.

Un tel masque comprend généralement une armature supportant un écran de protection transparent et une sangle fixée de part et d'autre de l'armature. La sangle permet de maintenir le masque sur le visage en entourant partiellement le crâne de l'utilisateur. Des éléments de confort peuvent être ajoutés à l'armature pour améliorer le confort du port du masque.

L'utilisateur peut souhaiter remplacer l'écran pour diverses raisons. Par exemple, il peut vouloir changer l'indice de filtration des rayonnements solaires, en fonction des conditions météorologiques. Il peut aussi remplacer un écran endommagé. Ce peut être aussi pour des raisons esthétiques, l'utilisateur voulant un écran correspondant aux dernières tendances de la mode.

Dans les masques connus, un écran de protection est le plus souvent inséré dans une fente périphérique ou localisée de l'armature du masque. Cette insertion est obtenue par une déformation élastique de l'écran et/ou de l'armature. Le maintien de l'écran est souvent assuré par des moyens d'encliquetage qui nécessitent également une déformation élastique de l'écran et/ou de l'armature. Ainsi, le remplacement de l'écran pour ce type de masque s'avère très délicat du fait qu'il demande une certaine force et dextérité. De plus, lors de cette opération, l'utilisateur est amené à toucher directement la surface de vision de l'écran. L'utilisateur peut donc rayer l'écran ou laisser ses empreintes sur la surface de vision. En conséquence, le champ de vision du masque peut être altéré.

Pour pallier ce problème, le brevet EP-B-1 810 648 propose un masque de protection oculaire comprenant un écran muni d'un rail supérieur. Ce rail est directement fixé sur l'écran et couvre toute la partie supérieure de celui-ci. La hauteur du rail ajoute une zone non transparente sur l'écran ce qui réduit le champ de vision. L'écran s'insère verticalement, vers le bas, dans une fente périphérique d'une armature. Pour maintenir l'écran dans la fente, c'est-à-dire, empêcher l'écran de remonter, le masque prévoit différents moyens de fixation de l'armature avec le rail, dans la partie médiane supérieure du masque. Cette conception permet d'éviter tout contact direct avec l'écran du fait que l'utilisateur peut saisir l'écran par le rail supérieur. Cependant, la préhension d'un tel écran ne s'avère pas très pratique car le rail est saisi par pincement, entre le pouce et l'index, sur un faible écartement : la largeur du rail. Cette proximité de contacts des doigts induit une prise qui ne se révèle pas très stable, l'écran pouvant pivoter autour des points de contact avec les doigts. Par ailleurs, l'insertion verticale de l'écran dans la fente de l'armature demande également une certaine dextérité. Le dimensionnement de l'écran et de l'armature doit ainsi être ajusté et tolère peu de variations dimensionnelles. Par exemple, si l'armature est un peu voilée, l'écran risque de ne pas facilement s'insérer dans la fente inférieure et cela peut gêner l'actionnement des moyens de fixation de l'écran.

Certains fabricants proposent des masques munis d'un écran intégrant un cadre rigide entourant complètement l'écran. La préhension de l'écran est améliorée. Cependant, la réalisation de ce type d'écran renforcé est complexe et coûteuse. Par ailleurs, on retrouve une problématique analogue pour fixer l'écran dans le cadre rigide. L'ensemble est également plus lourd du fait du cadre rigide additionnel.

La demande US 2011/0225709 décrit un masque à écran amovible fixé par un verrou rotatif. Ce verrou s'avère assez encombrant et complexe. Par ailleurs, la mise en place de l'écran demande un mouvement particulier de l'écran induisant une rotation qui peut être délicate à réaliser. Lors de l'assemblage, il est nécessaire de toucher l'écran ce qui peut salir l'écran. La demande US 2008/155736 décrit un autre masque à écran amovible. Le but de l'invention est de proposer un masque amélioré permettant l'interchangeabilité d'un écran.

Un autre but est notamment de faciliter le montage de l'écran sur l'armature du masque.

Un autre but est d'utiliser un écran simple et économique à réaliser.

Un but est de faciliter la préhension de l'écran.

Un autre but est d'avoir un masque plus agréable à porter et à utiliser.

L'invention est divulguée par la revendication indépendante 1 et propose un masque de protection oculaire pour la pratique de sport en plein air comprenant :
- un écran de protection oculaire,
- une armature périphérique délimitant une ouverture obturée par l'écran,
- des moyens d'accroche latéraux de l'écran sur l'armature périphérique permettant la solidarisation d'au moins une partie latérale de l'écran avec l'armature périphérique, les moyens d'accroche latéraux comprenant un verrou destiné à être en contact avec l'écran

Le masque est caractérisé par le fait que le verrou coulisse par rapport à l'armature périphérique selon une direction sensiblement horizontale.

Ainsi, cette conception de verrou offre une solution simple de solidarisation d'un écran avec une armature périphérique qui permet une mise en place très facile et rapide de l'écran sur l'armature périphérique.

Selon des aspects avantageux mais non obligatoires de l'invention, une telle fixation peut incorporer une ou plusieurs des caractéristiques suivantes, prises dans toute combinaison techniquement admissible :
- Chaque partie latérale de l'écran comprend une ouverture destinée à loger une saillie de positionnement de l'armature périphérique.
- Chaque saillie de positionnement de l'armature périphérique s'étend vers l'avant, depuis une surface d'appui contre laquelle s'appuie une partie de l'écran quand le masque est assemblé.
- L'armature périphérique comprend un logement pour les bords de l'écran, complètement ouvert vers l'avant du masque de sorte à permettre une mise en place de l'écran sur l'armature périphérique par simple translation selon une direction sensiblement perpendiculaire à l'ouverture.
- Le verrou se présente sous la forme d'un « U » de sorte que, en position verrouillage, une première paroi s'étend horizontalement devant l'écran et une deuxième paroi s'étend horizontalement derrière une partie de l'armature périphérique.
- Le verrou est guidé en translation par des moyens de guidage ménagés sur une boucle latérale fixée sur l'armature périphérique.
- Le masque comprend des moyens d'indexation permettant d'obtenir une position du verrou stable correspondant à la position verrouillée de l'écran.
- Le masque comprend deux verrous.
- L'écran de protection oculaire comporte un panneau optique, un premier insert de préhension fixé sur la partie supérieure du panneau optique et un deuxième insert de préhension fixé sur la partie inférieure du panneau optique. Selon une variante, les premier et deuxième inserts de préhension sont fixés sur la partie médiane du panneau optique. Alternativement, la largeur des premier et deuxième inserts de préhension est inférieure au tiers de la largeur du panneau optique. Selon un mode de réalisation, chaque insert de préhension comprend un logement ergonomique pour recevoir l'extrémité d'un doigt de la main.
- Au moins un insert de préhension constitue un moyen d'accroche de l'écran sur l'armature périphérique. Alternativement, au moins un insert de préhension comprend une saillie d'accroche apte à coopérer avec un logement ménagé sur l'armature périphérique de sorte que cette coopération permet de solidariser l'écran avec l'armature périphérique.

D'autres caractéristiques et avantages de l'invention seront mieux compris à l'aide de la description qui va suivre, en regard des dessins annexés illustrant, selon des formes de réalisation non limitatives, comment l'invention peut être réalisée, et dans lequel :

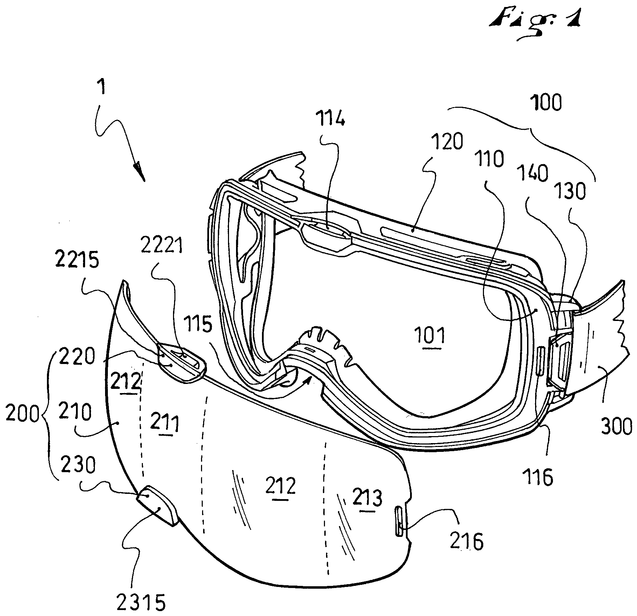

- la figure 1 est une vue en perspective avant éclatée d'un masque selon l'invention ;

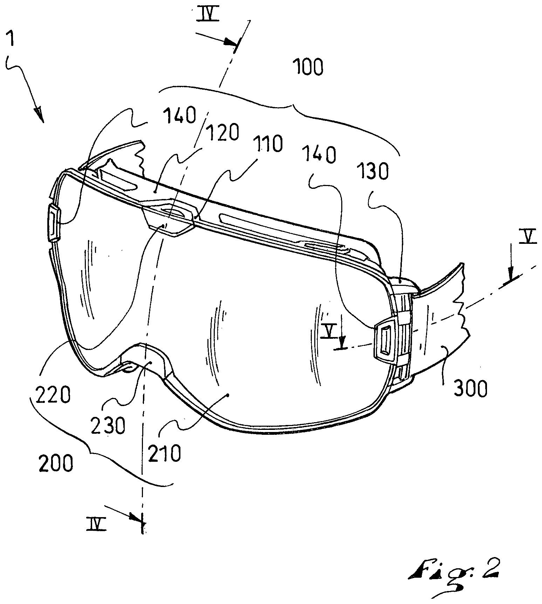

- la figure 2 est une vue en perspective avant du masque assemblé de la figure 1 ;
- la figure 3 est une vue en perspective arrière d'un écran du masque de la figure 1 manipulé par un utilisateur ;

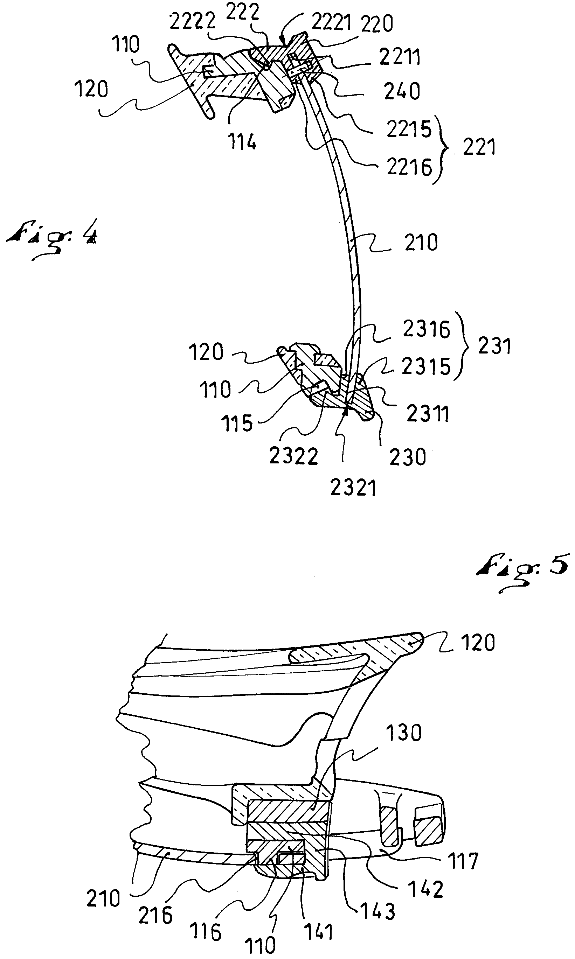

- la figure 4 est une section selon III-III de la figure 2 du masque assemblé de la figure 2;
- la figure 5 est une vue en coupe partielle selon IV-IV de la figure 2 du masque assemblé de la figure 2 ;
- la figure 6 est une vue en perspective éclatée partielle de l'armature du masque de la figure 1 ;
- les figures 7 à 9 sont des vues selon la direction F de la figure 6 d'éléments de l'armature du masque de la figure 1 illustrant un mode de réalisation du verrouillage de l'écran.

Le masque 1 comprend une armature périphérique 100. L'armature périphérique 100 délimite une ouverture 101. Cette ouverture 101 couvre, quand le masque est porté, une zone de vision s'étendant d'une tempe à l'autre, du front aux pommettes. L'ouverture 101 est obturée par un écran de protection oculaire 200. L'écran 200 est fixé à l'armature périphérique 100. L'écran 200 est assemblé sur l'armature périphérique 100 selon une direction horizontale, c'est-à-dire, selon une direction sensiblement perpendiculaire à l'ouverture 101. Une sangle 300 est fixée aux extrémités latérales de l'armature périphérique 100. La sangle 300 et l'armature périphérique 100 forment une boucle destinée à entourer le crâne de l'utilisateur pour assurer le maintien en place du masque.

Dans la suite de la description, il sera fait usage de termes tels que « horizontal », « vertical », « supérieur », « inférieur », « haut », « bas », « avant », « arrière », « devant », « derrière ». Ces termes doivent être interprétés en fait de façon relative en relation avec la position normale que le masque occupe sur le visage de l'utilisateur quand il se tient debout, tête droite. Par « arrière », on désigne les parties orientées côté tête / yeux de l'utilisateur.

L'écran de protection 200 obture l'ouverture 101 afin de protéger les yeux de l'utilisateur d'agressions extérieures telles que le vent, la neige ou l'eau. L'écran 200 comprend un panneau optique 210 et deux inserts de préhension 220, 230.

Le panneau optique 210 est transparent et peut présenter de façon connue en soi des propriétés de filtration lumineuse, afin d'assurer la protection des yeux de l'utilisateur contre le rayonnement solaire. Le panneau optique 210 pourra par exemple assurer la filtration des ultraviolets.

Le panneau optique 210 est habituellement réalisé en matière synthétique, par exemple, en polycarbonate ou acrylique. Il peut également être composé d'une ou plusieurs parois continues et transparentes disposées en vis-à-vis des yeux de l'utilisateur. Une paroi extérieure peut, par exemple, présenter des propriétés de filtrage des rayonnements solaires (par exemple pour réduire l'entrée des rayonnements ultraviolets dans le masque). Une paroi intérieure peut, par exemple, présenter des propriétés pour limiter la condensation de la vapeur d'eau.

Le panneau optique 210 présente deux zones de vision 212 destinées à venir en vis-à-vis des yeux de l'utilisateur. Les deux zones de vision 212 sont raccordées ensemble par une partie médiane 211. La partie médiane 211 est destinée à venir à l'aplomb du nez de l'utilisateur. De façon connue en soi, le panneau optique 210 peut former une paroi continue s'étendant entre les zones de vision 212 en passant par la partie médiane 211, afin d'optimiser l'angle de vision de l'utilisateur au niveau de la partie médiane. Le panneau optique 210 présente également des zones de vision périphérique 213 ménagées dans ses extrémités latérales.

A chacune des extrémités latérales, le panneau optique comprend une ouverture 216 s'étendant verticalement, selon une forme oblongue dans cet exemple.

Le panneau optique 210 est classiquement sphérique, c'est-à-dire, bombé horizontalement et verticalement mais peut également être cylindrique, voire plat. La bordure supérieure du panneau optique forme une ligne légèrement courbe. Les bordures latérales sont également légèrement courbes. La bordure inférieure suit un profil non linéaire. Le panneau optique est en effet évidé dans sa partie médiane inférieure pour le passage du nez. Cet évidement 214 forme un « V » inversé.

Un premier insert de préhension 220 est fixé sur la partie médiane supérieure du panneau optique. Un deuxième insert de préhension 230 est fixé sur la partie médiane inférieure du panneau optique, en vis-à-vis du premier insert. Dans cet exemple, le deuxième insert 230 comble le fond de l'évidement 214, et se localise sensiblement au niveau de l'os nasal quand le masque est porté.

Le premier insert 220 forme un « L » dont une première paroi 221 s'étend sensiblement verticalement vers le bas et dont une deuxième paroi 222 s'étend sensiblement horizontalement vers l'arrière. La première paroi 221 comprend une fente centrale 2211 destinée à recevoir une partie du panneau optique 210. Une partie de la face avant du panneau optique est alors recouvert par un voile avant 2215 et une partie de la face arrière du panneau optique est recouvert par un voile arrière 2216. Ce recouvrement partiel permet de saisir l'écran, par pincement selon l'épaisseur du panneau optique. Sur sa face supérieure, la deuxième paroi 222 comprend un logement ergonomique 2221, de forme arrondie, pour recevoir l'extrémité d'un doigt de la main. Sur sa face inférieure, la deuxième paroi 222 comprend une saillie d'accroche 2222 s'étendant vers le bas.

La largeur du premier insert 220 est inférieure au tiers de la largeur du panneau optique 210. Dans cet exemple, la largeur du panneau optique 210 est de vingt centimètres alors que la largeur de l'insert est d'environ trois centimètres. La largeur de l'insert est ainsi inférieure à 20% de la largeur du panneau optique. En étant de petite taille, l'insert de préhension peut facilement être réalisé pour un moindre coût. De plus, cela permet de concevoir un écran 200 très léger et procurant un champ de vision maximum.

Le premier insert 220 est centré sur la partie supérieure du panneau optique 210. Le panneau optique est introduit dans la fente centrale 2211 de la première paroi 221. Pour maintenir en position l'insert 220 sur le panneau optique 210, plusieurs solutions sont envisageables. Par exemple, ce peut être par collage, par serrage ou par goupillage. Dans le mode de réalisation illustré, les inserts sont assemblés grâce à des goupilles 240 se logeant dans des trous associés des inserts et du panneau optique.

Le deuxième insert 230 présente une structure analogue mais symétrique par rapport à un plan horizontal. Il forme un « L » dont une première paroi 231 s'étend sensiblement verticalement vers le haut et dont une deuxième paroi 232 s'étend sensiblement horizontalement vers l'arrière. La première paroi 231 comprend une fente centrale 2311 destinée à recevoir une partie du panneau optique 210. Une partie de la face avant du panneau optique est alors recouvert par un voile avant 2315 et une partie de la face arrière du panneau optique est recouvert par un voile arrière 2316. Sur sa face inférieure, la deuxième paroi 232 comprend un logement ergonomique 2321, de forme arrondie, pour recevoir l'extrémité d'un doigt de la main. Sur sa face supérieure, la deuxième paroi 232 comprend une saillie d'accroche 2322 s'étendant vers le bas.

La largeur du deuxième insert 230 est équivalente à celle du premier insert 220. Elle est inférieure à 20% de la largeur du panneau optique 210.

Le deuxième insert 230 est centré sur la partie inférieure du panneau optique 210. Le panneau optique est introduit dans la fente centrale 2311 de la première paroi 231. Les mêmes moyens d'assemblage que pour le premier insert sur le panneau optique peuvent être utilisés pour le deuxième insert.

Les deux inserts de préhension 220, 230 recouvrent chacun une partie de la tranche du panneau optique 210. De cette manière, lors de la saisie et la manipulation de l'écran, l'utilisateur ne touche pas le tranchant du panneau optique, le tranchant n'étant pas agréable au toucher.

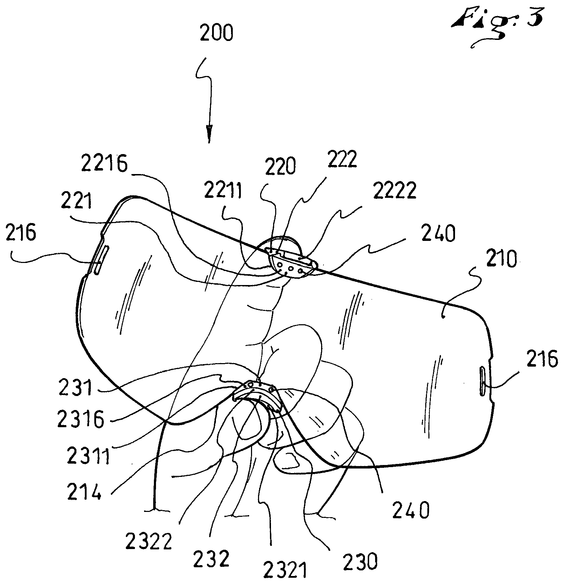

Grâce aux deux inserts de préhension 220 et 230, l'utilisateur peut facilement manipuler un écran en le prenant d'une manière stable comme on le voit à la figure 3. Par exemple, avec son pouce, l'utilisateur appuie sur un insert et avec son index, il appuie sur le deuxième insert permettant ainsi de pincer le masque sur sa hauteur entre deux doigts. Il s'ensuit une préhension très facile et stable. Ainsi, la saisie de l'écran peut être plus stable. L'utilisateur peut serrer l'écran entre son pouce et l'index en appuyant respectivement sur les premier et deuxième inserts de préhension. Du fait de la distance séparant ces deux inserts, c'est-à-dire, une partie de la hauteur de l'écran, la préhension est relativement stable. Il y a peu de risque de lâcher l'écran ou de toucher involontairement une surface de vision de l'écran. La manipulation de l'écran est simple et stable, ce qui facilite l'opération d'échange d'écran.

La prévision de seulement deux inserts de préhension, très localisés, permet de réduire au maximum les zones « obturées » de l'écran et permet donc d'optimiser le champ de vision, d'où un confort accru pour l'utilisateur.

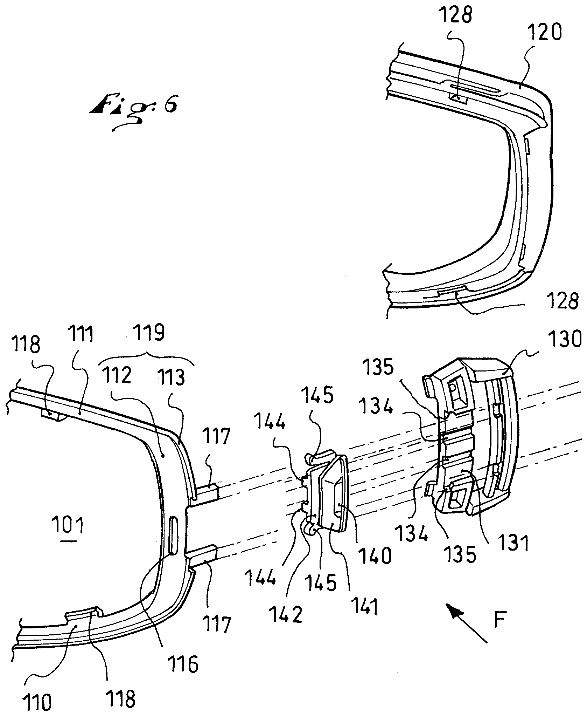

La figure 6 illustre la composition de l'armature périphérique 100. Elle comprend un cadre 110, une jupe 120, deux boucles latérales 130 et deux verrous latéraux 140. Pour améliorer le confort de portage, une mousse, non représentée, est fixée sur la partie interface avec le visage de l'utilisateur.

La jupe est réalisée dans un matériau plus souple que celui du cadre. La jupe permet de définir un volume intérieur du masque derrière l'écran, entre ce dernier et le visage de l'utilisateur. La jupe est destinée à pouvoir se déformer suffisamment pour s'adapter à la morphologie du visage de l'utilisateur ou à absorber des chocs appliqués sur le cadre. De manière classique, la jupe souple 120 est fixée sur le cadre rigide 110. Dans cet exemple, le cadre comprend des saillies 118, s'étendant vers l'arrière, réparties régulièrement sur la périphérie du cadre. Ces saillies 118 coopèrent avec des logements 128 de la jupe de sorte que la jupe 120 reste plaquée contre le cadre 110.

Le cadre 110 présente une bordure 111 reproduisant le contour du panneau optique 210, formant ainsi l'ouverture 101.

La bordure 111 comprend une surface d'appui 112 sous la forme d'une bande, de faible largeur, s'étendant verticalement et retraçant le contour du panneau. C'est contre cette surface d'appui 112 que l'écran 200 repose quand il est assemblé sur l'armature périphérique 100. Cette surface d'appui 112 constitue ainsi une butée ou un arrêt pour le déplacement vers l'arrière de l'écran 200.

D'autre part, la bordure 111 comprend un rebord 113 s'étendant horizontalement, vers l'avant du masque, depuis la surface d'appui 112. Ce rebord 113 reproduit le contour externe du panneau optique 210 avec une homothétie très légèrement supérieure à 1 de manière à ne pas être en contact avec la face interne du panneau optique 210 lorsque celui-ci est assemblé. Ce rebord 113 permet le positionnement vertical et latéral de l'écran 200 par rapport à l'armature périphérique 100. Il permet également le maintien vertical et latéral de l'écran.

Ainsi, la surface d'appui 112 et le rebord 113 forment un logement 119 pour les bords du panneau optique 210. La surface d'appui et/ou le rebord peuvent être continus sur toute la périphérie de la bordure 111 ou partiellement périphériques. Ce logement 119 est complètement ouvert vers l'avant du masque ce qui permet la mise en place directe de l'écran 200 sur l'armature périphérique 100, par simple translation horizontale, c'est-à-dire, selon une direction sensiblement perpendiculaire à l'ouverture 101. L'écran peut donc être positionné et monté très facilement dans le logement prévu sur la bordure 111 du cadre 110. Cet assemblage ne nécessite aucune inclinaison de l'écran, aucune insertion dans une fente, ni de déformation du panneau optique, ni même de clipsage du panneau optique. Pour ce mode de réalisation, il n'est pas besoin d'avoir un ajustement précis entre le contour externe du panneau d'optique 210 et le rebord 113. En conséquence, cette solution accepte des variations dimensionnelles des pièces constitutives du masque sans gêne pour l'assemblage de l'écran avec l'armature périphérique.

Pour maintenir l'écran 200 solidaire de l'armature périphérique 100, le dispositif de cet exemple comprend deux moyens d'accroche.

Le premier moyen d'accroche se compose des deux inserts de préhension 220, 230 et d'une partie du cadre rigide 110. Le cadre 110 comprend un premier logement 114 dans sa partie médiane supérieure. Ce premier logement 114 est dimensionné et positionné de manière à recevoir la saillie d'accroche 2222 du premier insert 220, lorsque l'écran 200 est mis en place sur l'armature périphérique 100. De même, un deuxième logement 115 est ménagé dans la partie médiane inférieure du cadre 110. Ce deuxième logement 115 est dimensionné et positionné de manière à recevoir la saillie d'accroche 2322 du deuxième insert 230, lorsque l'écran 200 avec l'armature périphérique 100. Le premier moyen d'accroche est donc un assemblage par clipsage, d'une part entre le premier insert 220 et la partie supérieure médiane du cadre 110 et, d'autre part, entre le deuxième insert 230 et la partie inférieure médiane du cadre 110.

Du fait de l'ouverture centrale 101, la bordure supérieure et la bordure inférieure du cadre 110 présentent une souplesse permettant à leur partie médiane de se déformer élastiquement selon une direction verticale. En conséquence, lorsque l'écran est mis en place sur l'armature périphérique, les deux saillies d'accroche 2222, 2322 provoquent la déformation verticale des bordures supérieure et inférieure, chacune se rapprochant du centre de l'ouverture 101. Puis, en poursuivant le déplacement de l'écran en direction de l'armature périphérique, les deux saillies d'accroche 2222, 2322 se placent en vis-à-vis des logements 114, 115. L'élasticité des bordures supérieure et inférieure suffit à les ramener dans leur position initiale. Dans cette configuration, les saillies d'accroche 2222, 2322 coopèrent respectivement avec leurs logements correspondant 114, 115. Cette coopération permet de maintenir la solidarisation de l'écran avec l'armature périphérique, notamment contre tout déplacement vers l'avant de l'écran 200.

Alternativement, ce peut être les deux saillies d'accroche 2222, 2322 qui se déforment pour une armature périphérique plus rigide.

Avantageusement, les inserts 220, 230 et le cadre 110 disposent de formes aménagées permettant d'aider les bordures du cadre à se déformer verticalement suite à un effort de rapprochement horizontal de l'écran vers l'armature périphérique. Ces aménagements sont par exemple des pentes ou arrondis.

Pour faciliter cette mise en place, le voile avant 2215, 2315 de chaque insert 220, 230 permet d'avoir une zone d'appui pour les doigts sans toucher l'écran. Ainsi, l'utilisateur peut appuyer sur l'écran pour le rapprocher de l'armature périphérique afin de faire coopérer les saillies d'accroche 2222, 2322 avec les logements 114, 115.

Ce premier moyen d'accroche par coopération de saillies d'accroche 2222, 2322 avec les logements 114, 115 est un moyen très simple, économique et léger. Ce premier moyen d'accroche permet l'assemblage de l'écran avec l'armature périphérique très facile et rapide. De plus, il permet une mise en place de l'écran selon une direction sensiblement horizontale ou, autrement dit, selon une direction sensiblement perpendiculaire à l'ouverture 101. Par « sensiblement », il faut comprendre plus ou moins 30° par rapport à l'horizontal.

Le deuxième moyen d'accroche assure la solidarisation des parties latérales de l'écran avec l'armature périphérique. Pour cela, le masque intègre deux mécanismes de verrouillage identiques, un pour chaque coté latéral du masque. Chaque mécanisme comprend une boucle latérale 130, un verrou latéral 140, une partie latérale du cadre 110 et une partie latérale de l'écran. Le mécanisme est illustré à travers les figures 6 à 9.

Dans ses parties latérales, la bordure 111 du cadre 110 comporte une surface d'appui 112 un peu plus large que pour le reste du contour. Cette surface d'appui 112, délimitant latéralement l'ouverture 101, s'étend sur toute la hauteur du cadre 110. A mi-hauteur, dans la partie centrale, une saillie de positionnement 116 s'étend vers l'avant, depuis la surface d'appui 112. La hauteur de cette saillie est sensiblement égale à l'épaisseur du panneau optique 210. Cette saillie de positionnement 116 prend une forme allongée orientée verticalement. Elle est destinée à se loger dans l'ouverture 216 du panneau optique quand l'écran est assemblé avec l'armature périphérique. En conséquence, la saillie 116 est dimensionnée et positionnée de sorte qu'elle puisse coopérer avec l'ouverture 216 quand le masque est assemblé. Cette coopération facilite le positionnement de l'écran sur l'armature périphérique et renforce le maintien vertical et latéral de l'écran.

Pour verrouiller latéralement l'écran, l'utilisateur fait coulisser latéralement un verrou 140. Ainsi, le verrou se translate relativement par rapport au cadre 110. Le mouvement est latéral ou sensiblement horizontal, l'orientation étant définie par une position normale d'utilisation du masque. Par « sensiblement », il faut comprendre plus ou moins 30° par rapport à l'horizontal.

Le verrou se présente sous la forme d'un « U » de sorte que, en position verrouillage, une première paroi 141 s'étend horizontalement devant le panneau optique 210 et une deuxième paroi 142 s'étend horizontalement derrière le cadre 110, les deux parois étant reliées par une jonction 143 s'étendant verticalement sur un côté de l'écran. Le masque comprend deux verrous, un pour chaque côté.

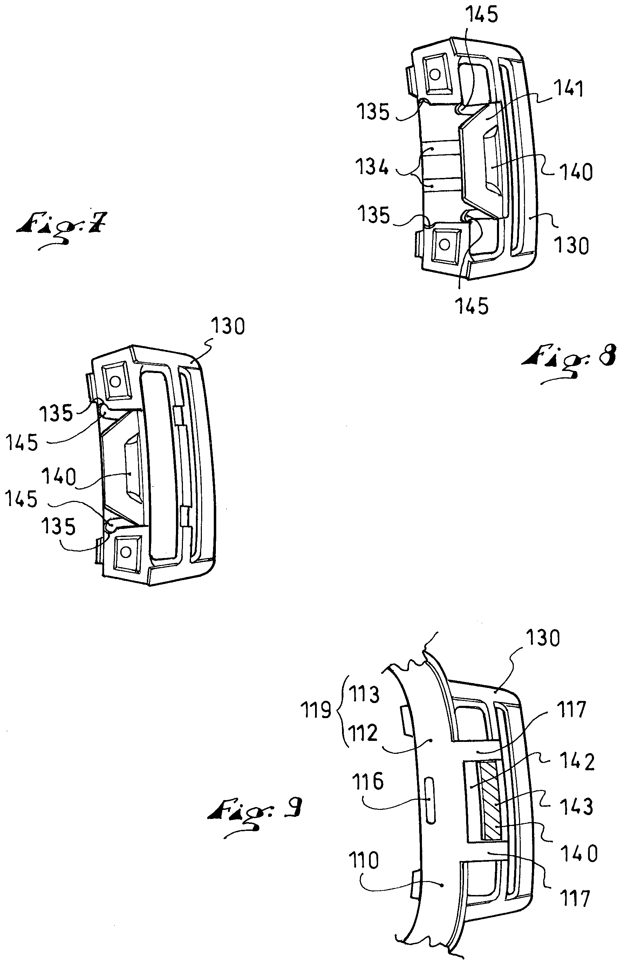

Lorsqu'un verrou est dans la configuration de verrouillage, il est positionné de manière qu'une partie latérale du cadre 110 et qu'une partie latérale du panneau optique 210 soient pris en sandwich entre les deux parois 141, 142 du verrou latéral 140. Dans cette configuration, représentée à la figure 7, non seulement la première paroi 141 couvre une partie latérale du panneau optique mais elle recouvre également l'ouverture 216 du panneau optique. Ainsi, l'écran se trouve complètement immobilisé par le verrou 140 coopérant avec le panneau optique 210 et le cadre 110. La présence de la première paroi 141 au-dessus de l'ouverture 216 assure le maintien de la saillie de positionnement 116 à l'intérieur de l'ouverture 216. Cette paroi 141 ferme alors le logement 119 du cadre 110 pour les bords du panneau optique 210, emprisonnant l'écran 200 dans ce logement 119. L'écran reste continuellement solidaire de cette saillie 116, c'est-à-dire, du cadre 110 et donc de l'armature périphérique 100. Cette liaison permet de conserver l'assemblage, même lorsque le masque fléchit autour d'un axe vertical. Il n'y a pas de risque que l'écran s'échappe d'une fente ou se déclippe. Par ailleurs, cette liaison, de chaque côté du panneau optique, permet un meilleur placage de l'écran vers le visage. En effet, lors de la mise en place du masque, la sangle 300 exerce un effort horizontal vers l'extérieur sur chaque partie latérale du cadre 110. Cela se traduit par un effort horizontal vers l'extérieur sur le panneau optique 210, via le contact entre les saillies 116 du cadre 110 avec les ouvertures correspondantes 216 du panneau optique 210. Ces liaisons, couplées avec la tension de la sangle suite à sa mise en place autour de la tête, génèrent donc une tension transversale/horizontale sur l'écran qui améliore le rapprochement du panneau optique vers les yeux. Le masque est plus agréable à porter, notamment grâce à un meilleur champ de vision apporté par ce positionnement de l'écran au plus près du visage.

Pour libérer l'écran, il suffit de faire coulisser chaque verrou horizontalement (latéralement), vers l'extérieur. Dans cette configuration de déverrouillage, représentée à la figure 8, chaque verrou est positionné de sorte que la première paroi 141 ne recouvre plus aucune partie du panneau optique 210. Cette action ouvre le logement 119 du cadre 110 pour les bords du panneau optique 210, libérant ainsi l'écran. Dès lors, on peut facilement retirer l'écran en le déplaçant vers l'avant. Il est à noter que, dans cette configuration, une partie de la deuxième paroi reste derrière la partie latérale du cadre 110 afin de garder chaque verrou accroché au masque. Cela permet d'éviter de perdre les verrous latéraux.

Dans cet exemple, l'armature périphérique 100 comprend, de chaque côté, une boucle latérale 130 fixée sur la partie latérale arrière du cadre 110, par des vis non représentées. Cette boucle latérale 130 sert, d'une part, d'attache pour une extrémité de la sangle 300 et, d'autre part, de guidage pour un verrou latéral 140.

La boucle latérale 130 comprend un évidement 131, ouvert vers l'avant, pour le passage de la deuxième paroi 142 du verrou 140, entre la boucle latérale 130 et le cadre 110. Dans cet évidement 131, la face orientée vers le cadre comprend deux rainures 134 s'étendant horizontalement. Ces rainures 134 sont destinées à coopérer avec deux saillies de guidage 144 disposées sur la face arrière de la deuxième paroi 142 du verrou 140. Cette coopération permet le guidage en translation latérale du verrou, notamment entre sa position de verrouillage et sa position de déverrouillage. Le verrou 140 coulisse donc selon une direction sensiblement horizontale par rapport à la boucle latérale 130 et, en conséquence, par rapport au cadre 110 du fait que la boucle latérale 130 est solidarisée avec le cadre 110.

Par ailleurs, les faces supérieure et inférieure de l'évidement 131 comprennent un logement 135 pour recevoir l'extrémité 145 de branches, déformables verticalement, ménagées sur le verrou latéral 140. Chaque verrou comprend une branche déformable supérieure et une branche déformable inférieure. Lorsque le verrou est dans sa configuration de verrouillage, chaque extrémité 145 d'une branche déformable coopère avec un logement 135 de la boucle latérale 130 correspondant. En conséquence, cette coopération permet d'obtenir une position du verrou 140 stable, selon une direction horizontale, ce qui permet de maintenir une configuration verrouillée de l'écran. Les extrémités 145 et les logements 135 forment donc des moyens d'indexation du verrou 140 par rapport à la boucle latérale 130 et donc au cadre 110. On évite alors tout risque de perdre l'écran lors de la pratique du sport. Lorsqu'on libère le verrou, l'utilisateur provoque le déplacement horizontal du verrou en exerçant un effort vers l'extérieur. Cette opération provoque le fléchissement des branches déformables ou clips. Les extrémités 145 sortent alors des logements 135.

Dans ce mode de réalisation, le guidage en translation latérale du verrou 140 a été renforcé par deux poutres 117 latéralement, vers l'extérieur, de la partie latérale du cadre 110. Ces deux poutres 117 sont écartées l'une de l'autre d'une distance légèrement supérieure à la hauteur de la jonction 143 du verrou latéral 140. La figure 9 représente une coupe partielle pour laquelle on a enlevé la première paroi 141 du verrou 140 afin de faciliter la compréhension de ce guidage. Ainsi, ce guidage de la jonction 143 par les deux poutres 117 complète le guidage en translation du verrou réalisé par les saillies de guidage 144 avec les rainures 134.

Ce deuxième moyen d'accroche présente l'avantage de ne nécessiter aucune déformation ou clipsage du masque mais également aucune déformation ou clipsage de l'armature périphérique, pour la solidarisation de l'écran avec l'armature périphérique.

Le mode de réalisation présenté décrit l'utilisation de deux moyens d'accroche. Alternativement, le masque peut en avoir seulement un. Par ailleurs, la solidarisation de l'écran avec l'armature périphérique peut être réalisée à l'aide d'autres mécanismes d'accroche que ceux détaillés dans la présente description. Par exemple, on peut envisager l'utilisation d'aimants pour accrocher l'écran avec l'armature périphérique. Un autre exemple consiste à utiliser un verrou bistable actionnable par pression sur le verrou.

De plus, le deuxième moyen d'accroche comprenant un verrou coulissant selon une direction latérale pourrait également s'appliquer à un masque doté d'un écran classique, par exemple, sans inserts de préhension.

Ce deuxième moyen d'accroche est une solution simple de solidarisation d'un écran avec une armature périphérique qui permet une mise en place très facile et rapide de l'écran sur l'armature périphérique.

Alternativement, les inserts de préhension peuvent avoir une forme différente.

## Revendications

1. Masque de protection oculaire (1) pour la pratique de sports en plein air comprenant :
- un écran de protection oculaire (200),
- un cadre (110) délimitant une ouverture (101) obturée par l'écran,
- des moyens d'accroche latéraux (110, 130, 140, 116, 216) de l'écran sur le cadre permettant la solidarisation d'au moins une extrémité latérale (213) de l'écran avec le cadre, les moyens d'accroche latéraux comprenant un verrou (140) destiné à recouvrir une partie de l'extrémité latérale de l'écran,
**caractérisé en ce que**
le verrou (140) coulisse par translation latérale par rapport au cadre entre une configuration
de verrouillage et une configuration de déverrouillage dans laquelle le verrou est positionné de sorte à ne plus recouvrir l'écran.

2. Masque de protection oculaire (1) selon la revendication 1, **caractérisé en ce que** chaque extrémité latérale de l'écran comprend une ouverture (216) destinée à loger une saillie de positionnement (116) du cadre.

3. Masque de protection oculaire (1) selon la revendication précédente, **caractérisé en ce que** chaque saillie de positionnement (116) du cadre s'étend vers l'avant, depuis une surface d'appui (112) contre laquelle s'appuie une partie de l'écran quand le masque est assemblé.

4. Masque de protection oculaire (1) selon l'une des revendications précédentes, **caractérisé en ce que** le cadre comprend un logement (119) pour les bords de l'écran, complètement ouvert vers l'avant du masque.

5. Masque de protection oculaire (1) selon l'une des revendications précédentes, **caractérisé en ce que** le verrou (140) se présente sous la forme d'un « U » de sorte que, en position verrouillage, une première paroi (141) s'étend horizontalement devant l'écran et une deuxième paroi (142) s'étend horizontalement derrière une partie du cadre.

6. Masque de protection oculaire (1) selon l'une des revendications précédentes, **caractérisé en ce que** le verrou (140) est guidé en translation par des moyens de guidage (134, 117) solidaire du cadre.

7. Masque de protection oculaire (1) selon l'une des revendications précédentes, **caractérisé en ce que** le masque comprend des moyens d'indexation (135, 145) permettant d'obtenir une position du verrou stable correspondant à la position verrouillée de l'écran.

8. Masque de protection oculaire (1) selon l'une des revendications précédentes, **caractérisé en ce que** le masque comprend une boucle latérale (130) fixée sur une partie latérale du cadre, le verrou (140) étant guidé en translation par la boucle latérale.

9. Masque de protection oculaire (1) selon l'une des revendications précédentes, **caractérisé en ce qu'**il comprend deux verrous (140).

10. Masque de protection oculaire (1) selon l'une des revendications précédentes, **caractérisé en ce que** l'écran de protection oculaire (200) comporte un panneau optique (210), un premier insert de préhension (220) fixé sur la partie supérieure du panneau optique et un deuxième insert de préhension (230) fixé sur la partie inférieure du panneau optique.

11. Masque de protection oculaire (1) selon la revendication précédente, **caractérisé en ce que** les premier et deuxième inserts de préhension sont fixés sur la partie médiane du panneau optique.

12. Masque de protection oculaire (1) selon l'une des revendications précédentes 10 à 11, **caractérisé en ce que** la largeur des premier et deuxième inserts de préhension est inférieure au tiers de la largeur du panneau optique.

13. Masque de protection oculaire (1) selon l'une des revendications précédentes 9 à 11, **caractérisé en ce que** chaque insert de préhension (220, 230) comprend un logement ergonomique (2221, 2321) pour recevoir l'extrémité d'un doigt de la main.

14. Masque de protection oculaire (1) selon l'une des revendications précédentes 9 à 12, **caractérisé en ce qu'**au moins un insert de préhension constitue un moyen d'accroche de l'écran sur le cadre.

15. Masque de protection oculaire (1) selon la revendication précédente, **caractérisé en ce qu'**au moins un insert de préhension comprend une saillie d'accroche (2222, 2322) apte à coopérer avec un logement (114, 115) ménagé sur le cadre de sorte que cette coopération permet de solidariser l'écran avec le cadre.

## Patentansprüche

1. Augenschutzbrille (1) zur Ausübung von Freiluftsport, die enthält:
- einen Augenschutzschirm (200),
- einen Rahmen (110), der eine vom Schirm verdeckte Öffnung (101) begrenzt,
- seitliche Befestigungseinrichtungen (110, 130, 140, 116, 216) des Schirms am Rahmen, die die feste Verbindung mindestens eines seitlichen Endes (213) des Schirms mit dem Rahmen ermöglichen, wobei die seitlichen Befestigungseinrichtungen einen Riegel (140) enthalten, der dazu bestimmt ist, einen Teil des seitlichen Endes des Schirms zu bedecken,
**dadurch gekennzeichnet, dass** der Riegel (140) durch seitliche Translation bezüglich des Rahmens zwischen einer Verriegelungskonfiguration und einer Entriegelungskonfiguration gleitet, in der der Riegel so positioniert ist, dass er den Schirm nicht mehr bedeckt.

2. Augenschutzbrille (1) nach Anspruch 1, **dadurch gekennzeichnet, dass** jedes seitliche Ende des Schirms eine Öffnung (216) enthält, die dazu bestimmt ist, einen Positioniervorsprung (116) des Rahmens aufzunehmen.

3. Augenschutzbrille (1) nach dem vorhergehenden Anspruch, **dadurch gekennzeichnet, dass** jeder Positioniervorsprung (116) des Rahmens sich von einer Auflagefläche (112) nach vorne erstreckt, gegen die sich ein Teil des Schirms anlegt, wenn die Brille zusammengebaut ist.

4. Augenschutzbrille (1) nach einem der vorhergehenden Ansprüche, **dadurch gekennzeichnet, dass** der Rahmen eine Aufnahme (119) für die Ränder des Schirms enthält, die zur Vorderseite der Brille hin vollständig offen ist.

5. Augenschutzbrille (1) nach einem der vorhergehenden Ansprüche, **dadurch gekennzeichnet, dass** der Riegel (140) die Form eines "U" hat, so dass in der Verriegelungsstellung eine erste Wand (141) sich waagrecht vor dem Schirm und eine zweite Wand (142) sich waagrecht hinter einem Teil des Rahmens erstreckt.

6. Augenschutzbrille (1) nach einem der vorhergehenden Ansprüche, **dadurch gekennzeichnet, dass** der Riegel (140) von fest mit dem Rahmen verbundenen Führungseinrichtungen (134, 117) in Translation geführt wird.

7. Augenschutzbrille (1) nach einem der vorhergehenden Ansprüche, **dadurch gekennzeichnet, dass** die Brille Indexierungseinrichtungen (135, 145) enthält, die es ermöglichen, eine stabile Stellung des Riegels zu erhalten, die der verriegelten Stellung des Schirms entspricht.

8. Augenschutzbrille (1) nach einem der vorhergehenden Ansprüche, **dadurch gekennzeichnet, dass** die Brille eine seitliche Öse (130) enthält, die an einem seitlichen Bereich des Rahmens befestigt ist, wobei der Riegel (140) von der seitlichen Öse in Translation geführt wird.

9. Augenschutzbrille (1) nach einem der vorhergehenden Ansprüche, **dadurch gekennzeichnet, dass** sie zwei Riegel (140) enthält.

10. Augenschutzbrille (1) nach einem der vorhergehenden Ansprüche, **dadurch gekennzeichnet, dass** der Augenschutzschirm (200) ein optisches Feld (210), einen ersten Greifeinsatz (220), der am oberen Teil des optischen Felds befestigt ist, und einen zweiten Greifeinsatz (230) aufweist, der am unteren Teil des optischen Felds befestigt ist.

11. Augenschutzbrille (1) nach dem vorhergehenden Anspruch, **dadurch gekennzeichnet, dass** die ersten und zweiten Greifeinsätze am Mittelteil des optischen Felds befestigt sind.

12. Augenschutzbrille (1) nach einem der vorhergehenden Ansprüche 10 bis 11, **dadurch gekennzeichnet, dass** die Breite der ersten und zweiten Greifeinsätze geringer als ein Drittel der Breite des optischen Felds ist.

13. Augenschutzbrille (1) nach einem der vorhergehenden Ansprüche 9 bis 11, **dadurch gekennzeichnet, dass** jeder Greifeinsatz (220, 230) eine ergonomische Aufnahme (2221, 2321) enthält, um das Ende eines Fingers der Hand aufzunehmen.

14. Augenschutzbrille (1) nach einem der vorhergehenden Ansprüche 9 bis 12, **dadurch gekennzeichnet, dass** mindestens ein Greifeinsatz eine Befestigungseinrichtung des Schirms am Rahmen bildet.

15. Augenschutzbrille (1) nach dem vorhergehenden Anspruch, **dadurch gekennzeichnet, dass** mindestens ein Greifeinsatz einen Befestigungsvorsprung (2222, 2322) enthält, der mit einer auf dem Rahmen ausgesparten Aufnahme (114, 115) so zusammenwirken kann, dass diese Zusammenwirkung die feste Verbindung des Schirms mit dem Rahmen ermöglicht.

## Claims

1. Protective goggles (1) for outdoor sports, comprising:
- a protective optical shield (200),
- a frame (110) delimiting an opening (101) which is closed by the shield,
- lateral means (110, 130, 140, 116, 216) for latching the shield to the frame, allowing at least one lateral end (213) of the shield to be secured to the frame, the lateral latching means comprising a lock (140) that is designed to cover part of the lateral end of the shield,
**characterized in that**
the lock (140) slides by lateral translation, with respect to the frame, between a locked configuration and an unlocked configuration in which the lock is positioned so that it no longer covers the shield.

2. Protective goggles (1) according to Claim 1, **characterized in that** each lateral end of the shield comprises an opening (216) for receiving a positioning projection (116) of the frame.

3. Protective goggles (1) according to the preceding claim, **characterized in that** each positioning projection (116) of the frame extends forwards, from a bearing surface (112) against which part of the shield presses when the goggles are assembled.

4. Protective goggles (1) according to one of the preceding claims, **characterized in that** the frame comprises a recess (119) for the edges of the shield, this recess being fully open to the front of the goggles.

5. Protective goggles (1) according to one of the preceding claims, **characterized in that** the lock (140) is U-shaped such that, in the locked position, a first wall (141) extends horizontally in front of the shield and a second wall (142) extends horizontally behind part of the frame.

6. Protective goggles (1) according to one of the preceding claims, **characterized in that** the lock (140) is guided in translation by guide means (134, 117) that are fixed to the frame.

7. Protective goggles (1) according to one of the preceding claims, **characterized in that** the goggles comprise indexing means (135, 145) with which it is possible to obtain a stable position of the lock corresponding to the locked position of the shield.

8. Protective goggles (1) according to one of the preceding claims, **characterized in that** the goggles comprise a lateral loop (130) attached to a lateral part of the frame, the lock (140) being guided in translation by the lateral loop.

9. Protective goggles (1) according to one of the preceding claims, **characterized in that** it comprises two locks (140).

10. Protective goggles (1) according to one of the preceding claims, **characterized in that** the protective optical shield (200) comprises an optical panel (210), a first gripping insert (220) attached to the upper part of the optical panel, and a second gripping insert (230) attached to the lower part of the optical panel.

11. Protective goggles (1) according to the preceding claim, **characterized in that** the first and second gripping inserts are attached to the middle part of the optical panel.

12. Protective goggles (1) according to one of the preceding Claims 10 and 11, **characterized in that** the width of the first and second gripping inserts is less than one-third of the width of the optical panel.

13. Protective goggles (1) according to one of the preceding Claims 9 to 11, **characterized in that** each gripping insert (220, 230) comprises an ergonomic recess (2221, 2321) for receiving a fingertip.

14. Protective goggles (1) according to one of the preceding Claims 9 to 12, **characterized in that** at least one gripping insert constitutes a means for latching the shield onto the frame.

15. Protective goggles (1) according to the preceding claim, **characterized in that** at least one gripping insert comprises a latching projection (2222, 2322) for engaging with a recess (114, 115) created on the frame such that, by this engagement, the shield can be secured to the frame.
